Europäisches Patentamt

European Patent Office

Office européen des brevets

(19)

(11) Numéro de publication : **0 245 142 B1**

(12)                    **FASCICULE DE BREVET EUROPEEN**

(45) Date de publication du fascicule du brevet :
**13.03.91 Bulletin 91/11**

(51) Int. Cl.⁵ : **A61M 16/04**

(21) Numéro de dépôt : **87400888.1**

(22) Date de dépôt : **17.04.87**

(54) **Tube pour assistance respiratoire.**

(30) Priorité : **29.04.86 FR 8606185**
**16.12.86 FR 8617541**

(43) Date de publication de la demande :
**11.11.87 Bulletin 87/46**

(45) Mention de la délivrance du brevet :
**13.03.91 Bulletin 91/11**

(84) Etats contractants désignés :
**AT BE CH DE ES FR GB GR IT LI LU NL SE**

(56) Documents cités :
**US-A- 3 881 479**

(73) Titulaire : **Boussignac, Georges**
**1 avenue de Provence**
**F-92160 Antony (FR)**
Titulaire : **Labrune, Jean-Claude**
**2, avenue de Guyenne**
**F-92160 Antony (FR)**

(72) Inventeur : **Boussignac, Georges**
**1 avenue de Provence**
**F-92160 Antony (FR)**
Inventeur : **Labrune, Jean-Claude**
**2, avenue de Guyenne**
**F-92160 Antony (FR)**

(74) Mandataire : **Bonnetat, Christian et al**
**CABINET BONNETAT 23, Rue de Léningrad**
**F-75008 Paris (FR)**

## Description

La présente invention a pour objet un tube pour assistance respiratoire, utilisable sur des patients dont la respiration spontanée est absente ou insuffisante, qu'ils soient placés ou non sous respiration artificielle.

On connaît divers dispositifs, tels que des sondes ou des canules orales, nasales, endotrachéales, trachéotomiques, destinés à faire la jonction entre un appareil de respiration artificielle et/ou d'anesthésie et le système respiratoire d'un patient.

Ces dispositifs, essentiellement en forme de tubes, peuvent, selon le cas, comporter des moyens d'immobilisation, tels que des pattes ou des collerettes au voisinage de l'extrémité proximale, pour le maintien sur la bouche ou le nez du patient, ou encore des ballonnets gonflables au voisinage de l'extrémité distale, pour le maintien par friction dans la trachée.

Ces dispositifs connus donnent satisfaction dans la pratique courante, mais présentent toutefois des inconvénients.

Ainsi, par exemple, lorsqu'un tube de type connu est déconnecté du respirateur artificiel et que le patient a besoin d'air enrichi en oxygène, il est nécessaire d'introduire dans ledit tube une sonde reliée à une source d'oxygène.

Par ailleurs, dans les cas de respiration spontanée insuffisante, le patient doit nécessairement rester relié au respirateur jusqu'au rétablissement complet de sa respiration spontanée.

C'est pourquoi on connaît également des tubes comportant un canal auxiliaire, parallèle au canal principal et débouchant à l'extrémité distale, pour la ventilation par injection d'air et/ou d'oxygène. Mais ces tubes à canaux auxiliaires présentent à leur tour des inconvénients du fait que le jet auxiliaire d'air et/ou d'oxygène vient frapper directement la muqueuse, d'où des risques non négligeables de traumatismes.

Pour remédier à cet inconvénient, le brevet américain US-A-3 881 479 décrit un tube d'assistance respiratoire pourvu d'un canal auxiliaire à oxygène débouchant à l'intérieur d'un canal principal, à faible distance de l'extrémité distale de celui-ci. Ainsi, l'oxygène débouche dans le canal principal et y provoque des turbulances de sorte que le patient, auquel ce tube d'assistance respiratoire est appliqué, inspire de l'oxygène assagi à travers le canal auxiliaire et l'extrémité distale du canal principal. Il expire à travers le canal principal. Toutefois, avec ce type de tube d'assistance respiratoire, on peut rencontrer des difficultés concernant la circulation des gaz dans le canal principal.

La présente invention a pour objet de perfectionner un tube d'assistance respiratoire de ce type en permettant notamment de contrôler le passage de gaz à travers le canal principal.

A cette fin, selon l'invention, le tube pour assistance respiratoire comportant un canal principal et au moins un canal auxiliaire parallèle au canal principal et débouchant à l'intérieur du canal principal à faible distance de l'extrémité distale, est remarquable en ce que ledit canal principal comporte, à proximité de l'orifice de sortie du canal auxiliaire, un moyen formant venturi.

Suivant que l'on veut favoriser l'inspiration ou l'expiration du patient, ledit canal auxiliaire débouche en aval ou en amont dudit moyen formant venturi. Ce dernier peut être réalisé de toute façon connue, par exemple par rétrécissement de forme appropriée de la section interne du canal principal ou par insertion d'une bague à l'intérieur de ce dernier.

On peut prévoir au moins un canal auxiliaire débouchant en aval et au moins un canal auxiliaire débouchant en amont du moyen formant venturi.

De plus, pour alimenter séparément les deux poumons d'un patient, le tube selon l'invention peut être dédoublé, au voisinage de son extrémité distale, en deux canaux principaux contenant chacun au moins un canal auxiliaire.

Pour commander encore mieux le passage gazeux à travers le canal principal, le tube respiratoire selon l'invention comporte avantageusement au moins un canal auxiliaire supplémentaire débouchant à l'intérieur du canal principal à faible distance de l'extrémité proximale et un moyen formant venturi est disposé à proximité de l'orifice de sortie dudit canal auxiliaire supplémentaire.

Grâce à l'invention, il devient possible dans certains cas de se passer d'un respirateur artificiel et, dans de nombreux cas, d'en réduire la durée d'utilisation. Il s'agit là d'un avantage essentiel car, comme cela est bien connu, la mise en oeuvre des respirateurs artificiels relève d'une très haute compétence des opérateurs et, même utilisé selon les règles de l'art, un respirateur ne respecte pas les critères de la respiration spontanée. En effet, lors de la phase d'inspiration, la pression risque souvent d'être telle que les capillaires pulmonaires sont écrasés, c'est-à-dire que la circulation sanguine pulmonaire est bloquée au moment même où l'échange d'oxygène devrait avoir lieu.

La possibilité dans certains cas de réduire la durée d'utilisation du respirateur artificiel n'est pas seulement avantageuse parce qu'elle permet d'en éviter les dangers, mais également parce qu'elle permet d'en éviter les frais (coût de l'appareil et de son entretien, coût de la main d'oeuvre) et l'encombrement (application aux secours urgents).

Une autre difficulté soulevée par la respiration artificielle est celle de l'espace mort ("closing volume"), c'est-à-dire du volume gazeux ventilé qui ne participe pas aux échanges avec le sang, et dont une fraction importante peut être due à l'appareillage: il s'agit dans ce cas du volume de la zone balayée alternativement par le gaz insufflé puis exsufflé, zone dans laquelle subsiste donc une partie du gaz exsufflé chargé de gaz carbonique et qui est réinsufflée à l'insufflation suivante. Grâce à sa structure, le tube d'assistance respiratoire selon l'invention permet de réduire autant que possible cet espace mort, ce qui est surtout important dans le cas d'un volume de ventilation globale faible, comme chez les jeunes enfants et les nourrissons porteurs de membranes hyalines.

Ces problèmes de la ventilation artificielle, ainsi que d'autres, sont bien décrits par F. Fleur et I. Saint-Jean dans Mat. Méd. Chir., 181, 1, 4-12.

Les figures du dessin annexé feront bien comprendre comment l'invention peut être réalisée.

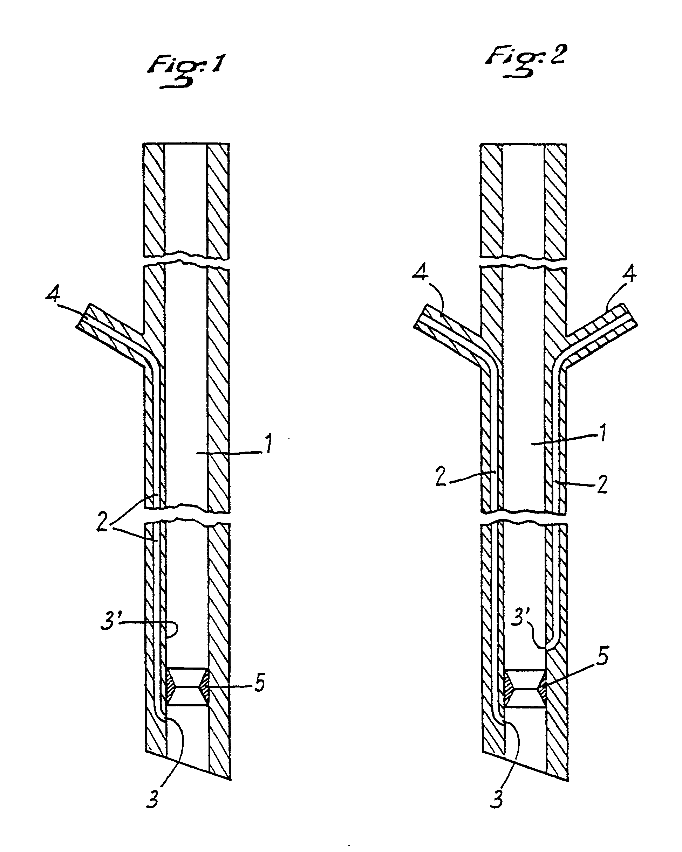

Les figures 1 à 4 sont des vues schématiques en coupe longitudinale de différents modes de réalisation du tube d'assistance respiratoire conforme à la présente invention. Sur ces figures, on a représenté ces tubes sous forme rectiligne. Il va de soi, en réalité, que ces tubes sont souples comme celui décrit dans le brevet américain précité ou que, selon le conduit naturel par lequel le tube est destiné à être mis en place, il est plus ou moins courbé, ou cintré, pour traumatiser le patient le moins possible. Le tube selon l'invention peut se présenter sous la forme d'appareils connus, tels que sonde endotrachéale oronasale avec ou sans ballonnet, sonde endotrachéale pédiatrique, sonde de monitorage des gaz, sonde endobronchique, sonde nasopharyngée, sonde d'intubation anatomique pour enfant, sonde de Cole néonatale, sonde canule de Guedel ou sonde nasale d'oxygénothérapie.

Dans la présente description, on appelle extrémité proximale l'extrémité du tube restant au dehors du patient et pouvant être reliée par exemple à un respirateur artificiel ; on appelle extrémité distale l'extrémité du tube se trouvant, en position d'utilisation à l'intérieur du corps du patient. De même, on appelle sens amont le sens qui va d'un point donné vers l'extrémité proximale, et sens aval le sens qui va d'un point donné vers l'extrémité distale.

Le tube illustré schématiquement sur la figure 1 comporte d'une part, un canal principal 1 s'étendant de l'extrémité proximale (haut de la figure) jusqu'à l'extrémité distale (bas de la figure), et, d'autre part, un canal auxiliaire 2 débouchant en 3 à l'intérieur du canal principal et à faible distance de l'extrémité distale. Ce canal auxiliaire 2 peut présenter un diamètre de quelques centaines de microns.

L'extrémité proximale 4 du canal auxiliaire 2 est extérieure au tube proprement dit. Elle consiste en un embout destiné à être relié à une source de gaz respirable, tel qu'un mélange d'oxygène et d'air.

Le tube étant en place sur un patient, l'insufflation du gaz par le canal auxiliaire 2 amène ledit gaz directement à l'entrée des bronches, diminuant ainsi considérablement l'espace mort.

Sur la plus grande partie de son trajet, le canal auxiliaire 2 court parallèlement au canal 1. Il peut être constitué d'un tube de faible diamètre, solidaire du tube principal, soit à l'extérieur de ce dernier et traversant sa paroi au voisinage 3 de l'extrémité distale, soit à l'intérieur et traversant donc ladite paroi à hauteur de l'embout 4. Mais de préférence, et comme représenté sur la figure, le canal 2 est incorporé dans la paroi du tube, de sorte que les sections droites internes et externes de ce dernier soient parfaitement uniformes, par exemple parfaitement circulaires.

Le canal principal 1 et le canal auxiliaire 2 pourraient être coaxiaux, au moins au voisinage de l'extrémité distale, l'orifice 3 du tube auxiliaire coaxial se situant alors dans l'axe du canal principal 1 et à faible distance en amont de son extrémité distale.

Conformément à l'invention, à l'intérieur du canal 1 et au voisinage de l'orifice 3, est disposée et fixée une bague 5, dont le diamètre extérieur est sensiblement égal au diamètre du canal 1, et dont la variation de section forme un venturi.

Ainsi, lorsque du gaz respirable est injecté à pression élevée (plusieurs bars) par l'embout 4 à travers le canal auxiliaire 2, ce gaz débouche à grande vitesse par l'orifice 3 dans le canal 1 et y provoque l'entraînement du fluide dans le canal principal 1, sous la dépendance du venturi formé par la bague 5. Ainsi, la respiration du patient est grandement favorisée.

Dans la position montrée sur la figure 1, c'est-à-dire lorsque l'orifice 3 se trouve en aval de la bague 5, l'inspiration du patient est favorisée. En revanche, si le canal 2 débouchait en un point 3' situé en amont de la bague 5, c'est l'expiration du patient qui serait favorisée.

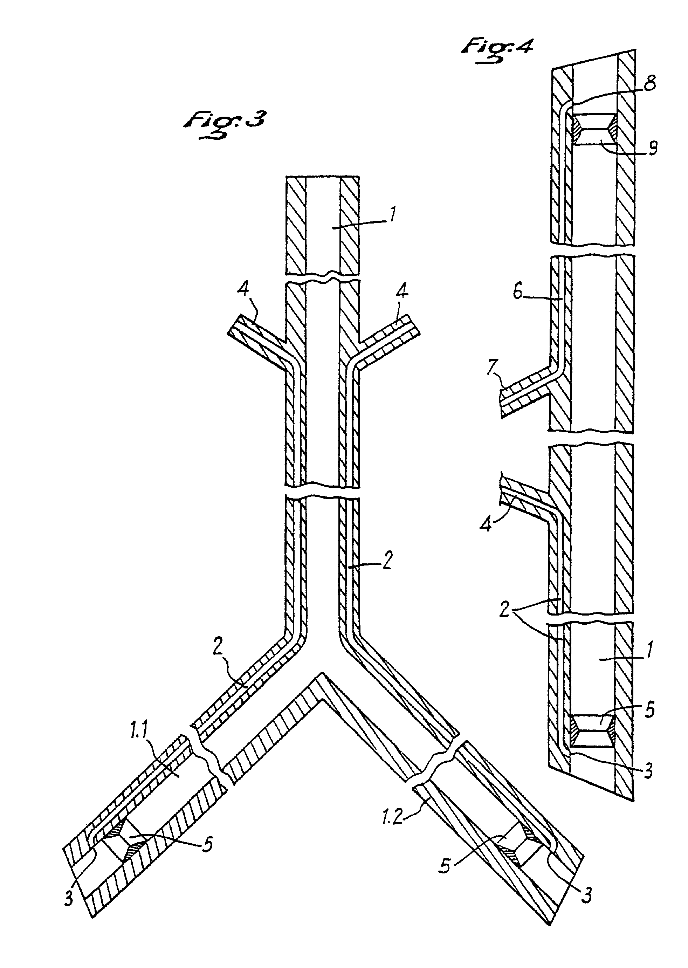

On peut prévoir, comme cela est représenté sur la figure 2, le long du canal principal 1, au moins un canal auxiliaire 2 débouchant en aval (en 3), et au moins un canal 2 débouchant en amont (en 3') de la bague 5 formant venturi, les embouts 4 de chacun des canaux 2 pouvant être connectés alternativement à une source de gaz respirable, permettant ainsi de favoriser respectivement l'inspiration et l'expiration du patient.

Un dispositif constitué d'un tel tube à deux canaux auxiliaires 2, d'une source de gaz respirable et d'un système de commutation sera donc moins encombrant, moins coûteux et d'un maniement plus simple qu'un respirateur artificiel, dans les cas où il peut remplacer ce dernier.

Dans le cas où un patient non seulement présente une insuffisance respiratoire, mais en plus ne présente plus la "symétrie" qui existe normalement entre les deux poumons, il faut alimenter séparément chaque poumon, ce qui, avec la technique connue, peut impliquer la mise en oeuvre de deux respirateurs artificiels synchronisés. C'est pourquoi, et conformément à la variante de réalisation de la figure 3, le tube d'assistance respiratoire peut être dédoublé au voisinage de son extrémité distale, l'ensemble présentant alors la forme générale d'un Y renversé. Dans le tronc d'un tel tube en Y passe donc un canal principal 1 et au moins deux canaux auxiliaires 2, le canal principal 1 se partageant en deux canaux 1.1 et 1.2 dont chacun passe dans une branche du Y, et chacun des canaux auxiliaires 2 passant d'abord le long du tronc puis le long de l'une ou de l'autre des branches.

Dans la variante de réalisation de l'invention monté par la figure 4, le canal principal 1 s'étend de l'extrémité proximale (haut de la figure) jusqu'à l'extrémité distale (bas de la figure), et on prévoit au moins deux canaux auxiliaires 2 et 6, dont l'un 2 débouche en 3 à l'intérieur du canal 1 et à faible distance de l'extrémité distale, et l'autre débouche en 8 à l'intérieur du canal 1 et à faible distance de l'extrémité proximale. Chacun des canaux auxiliaires 2 et 6 peut être raccordé au milieu extérieur grâce à un embout, respectivement 4 et 7, embouts destinés à être reliés à une source de gaz respirable telle qu'un mélange d'oxygène et d'air.

Le tube étant en place sur un patient, l'insufflation de gaz se fait alternativement dans le canal 2 (par l'embout 4) et dans le canal 6 (par l'embout 7), de manière à favoriser alternativement l'inspiration et l'expiration du patient. A cet effet un dispositif (non représenté) de commutation et de réglage des débits et des durées d'insufflation de gaz est relié aux embouts 4 et 7, d'une part, et à la source de gaz, d'autre part.

Par réglage adéquat, il devient possible de maintenir, grâce à un canal auxiliaire de type 2, une pression positive dans les poumons du patient, et cela pendant ou à la fin de la phase expiratoire provoquée ou assistée par l'insufflation de gaz dans l'autre canal auxiliaire 6, de façon à supprimer tout risque de collapsus alvéolaire.

Dans les canaux auxiliaires 2 et 6, sont prévues des bagues 5 et 9, respectivement, formant venturi et disposées à proximité des extrémités internes respectives 3, 8 desdits tubes auxiliaires.

Par analogie au mode de réalisation de la figure 3, l'extrémité distale du tube peut être dédoublée en vue d'alimenter différemment chacun des deux poumons. L'ensemble présente alors la forme générale d'un Y renversé. Un tel tube en Y comprendra donc au moins trois canaux auxiliaires, l'un débouchant au voisinage de l'extrémité proximale, chacun des deux autres débouchant au voisinage de l'une des deux extrémités distales.

Quelle que soit la variante de réalisation du tube selon l'invention, il peut comporter évidemment des moyens de fixation tels que ceux dont sont munis les tubes de types connus : pattes ou collerettes au voisinage de l'extrémité proximale, ballonnets gonflables au voisinage de l'extrémité distale.

Les matériaux constitutifs du tube selon l'invention sont également ceux des tubes de types connus: il s'agit essentiellement de polychlorure de vinyle, avec éventuellement revêtement de silicone.

Enfin les dimensions de l'ensemble peuvent être très variables, essentiellement selon la voie de mise en place du tube, et selon la taille du patient qui peut être un adulte, un enfant ou un nourrisson.

## Revendications

1. Tube pour assistance respiratoire, comportant un canal principal (1) et au moins un canal auxiliaire (2) parallèle au canal principal et débouchant à l'intérieur du canal principal à faible distance (3) de l'extrémité distale, caractérisé en ce que ledit canal principal (1) comporte, à proximité de l'orifice de sortie (3) du canal auxiliaire (2), un moyen (5) formant venturi.

2. Tube selon la revendication 1, caractérisé en ce que le canal auxiliaire (2) débouche en aval du moyen (5) formant venturi.

3. Tube selon la revendication 1, caractérisé en ce que le canal auxiliaire (2) débouche en amont du moyen (5) formant venturi.

4. Tube selon la revendication 1, caractérisé en ce qu'il comporte au moins un canal auxiliaire (2) débouchant en aval et au moins un canal auxiliaire (2) débouchant en amont du moyen (5) formant venturi.

5. Tube selon la revendication 1, caractérisé en ce que, au voisinage de son extrémité distale, il est dédoublé en deux canaux principaux (1) contenant chacun au moins un canal auxiliaire (2).

6. Tube selon la revendication 1, caractérisé en ce qu'il comprend au moins un canal auxiliaire supplémentaire (6) débouchant à l'intérieur du canal principal (1) à faible distance (8) de l'extrémité proximale et en ce qu'un moyen (9) formant venturi est disposé à proximité de l'orifice de sortie dudit canal auxiliaire supplémentaire.

7. Tube selon l'une des revendications 1 ou 6, caractérisé en ce que le ou les canaux auxiliaires (2, 6) sont incorporés dans la paroi du canal principal (1).

## Ansprüche

1. Kanüle zur Beatmungsunterstützung, welche einen Hauptkanal (1) und mindestens einen Hilfskanal (2) aufweist, der parallel zum Hauptkanal verläuft und mit geringem Abstand (3) zum distalen Ende ins Innere des Hauptkanals mündet, dadurch gekennzeichnet, daß der genannte Hauptkanal (1), in der Nähe der Ausgangsmündung (3) des Hilfskanals (2), eine eine Venturidüse bildende Einrichtung (5) aufweist.

2. Kanüle nach Anspruch 1, dadurch gekennzeichnet, daß der Hilfskanal (2) stromabwärts der die Venturidüse bildenden Einrichtung (5) mündet.

3. Kanüle nach Anspruch 1, dadurch gekennzeichnet, daß der Hilfskanal (2) stromaufwärts der die Venturidüse bildenden Einrichtung (5) mündet.

4. Kanüle nach Anspruch 1, dadurch gekennzeichnet, daß sie mindestens einen stromabwärts der die Venturidüse bildenden Einrichtung (5) mündenden Hilfskanal (2) und mindestens einen stromaufwärts der die Venturidüse bildenden Einrichtung (5) mündenden Hilfskanal (2) aufweist.

5. Kanüle nach Anspruch 1, dadurch gekennzeichnet, daß sie nahe ihrem distalen Ende in zwei Hauptkanäle (1) aufgespalten ist, wobei jeder mindestens einen Hilfskanal (2) aufweist.

6. Kanüle nach Anspruch 1, dadurch gekennzeichnet, daß sie mindestens einen zusätzlichen Hilfskanal (6) aufweist, welcher mit geringem Abstand (B) zum proximalen Ende ins Innere des Hauptkanals (1) mündet, und daß eine eine Venturidüse bildende Einrichtung (9) in der Nähe der Ausgangsmündung des genannten zusätzlichen Hilfskanals angeordnet ist.

7. Kanüle nach einem der Ansprüche 1 oder 6, dadurch gekennzeichnet, daß der oder die Hilfskanäle (2, 6) in der Wandung des Hauptkanals (1) untergebracht ist/sind.

## Claims

1. Breathing aid tube, having a main channel (1) and at least one auxiliary channel (2) parallel to the main channel and emerging inside the main channel at a small distance (3) from the distal end, characterized in that said main channel (1) has a venturi forming means (5) in the vicinity of the outlet orifice (3) of the auxiliary channel (2).

2. Tube according to claim 1, characterized in that said auxiliary channel (2) emerges downstream of said venturi forming means (5).

3. Tube according to claim 1, characterized in that said auxiliary channel (2) emerges upstream of said venturi forming means (5).

4. Tube according to claim 1, characterized in that it comprises at least one auxiliary channel (2) emerging downstream and at least one auxiliary channel (2') emerging upstream of said venturi forming means (5).

5. Tube according to claim 1, characterized in that it is split, in the vicinity of its distal end, into two main channels (1) each containing at least one auxiliary channel (2).

6. Tube according to claim 1, characterized in that it includes at least one additional auxiliary channel (6) emerging inside said main channel (1) at a small distance (8) from the proximal end and in that a venturi forming means (9) is disposed in the vicinity of the outlet orifice of said additional auxiliary channel.

7. Tube according to one of claims 1 or 6, characterized in that said auxiliary channel or channels (2, 6) are incorporated in the wall of said main channel (1).

*Fig.1*

*Fig.2*

4

1

2

3'

5

3

4

4

1

2

2

3'

5

3

Fig.4

Fig.3